# EUROPEAN PATENT APPLICATION

(11) **EP 4 403 102 A1**
(43) Date of publication of application: **24.07.2024**
(21) Application number: 22870279.1
(22) Date of filing: 14.09.2022
(51) Int. Cl.: A61B 5/024, A61B 5/00, G16H 50/20

(54) **DEEP LEARNING-BASED ATRIAL FIBRILLATION DETERMINATION SYSTEM USING PPG SIGNAL DETECTION RING**

(30) Priority: 16.09.2021 KR 20210124273
(71) Applicant: Sky Labs Inc., Seongnam-si, Gyeonggi-do 13486 (KR)
(72) Inventor: CHANG, Hyung Min, Yongin-si, Gyeonggi-do 16843 (KR); KIM, Hae Na, Seongnam-si, Gyeonggi-do 13466 (KR); JO, Sung Mi, Seoul 08706 (KR); LEE, Min Hyung, Bucheon-si, Gyeonggi-do 14600 (KR); KIM, Chang Hyun, Seoul 05372 (KR); CHOI, Chang Woo, Uiwang-si, Gyeonggi-do 16000 (KR); LEE, Byung Hwan, Yongin-si, Gyeonggi-do 16981 (KR)
(74) Representative: Loyer & Abello
(86) International application number: PCT/KR2022/013734
(87) International publication number: WO 2023/043195

(57) **Abstract**

A deep-learning-based atrial fibrillation determination system using a photoplethysmography (PPG) signal detection ring is provided. The system may include a server, the server may include a signal quality classification component configured to classify the quality of a PPG signal as good or bad by using a first deep learning model, and an atrial fibrillation determination component configured to determine, by using a second deep learning model, from the PPG signal, whether atrial fibrillation has occurred, wherein the PPG signal detection ring includes a plurality of sensors configured to simultaneously measure a plurality of PPG signals at different locations, each of the plurality of sensors includes a light source and a photoelectric conversion device, the PPG signal is measured using the PPG signal detection ring, the server receives the PPG signal from the PPG signal detection ring through a terminal, and the terminal includes a sensor selection component configured to select, from among the plurality of sensors, as a sensor for measuring the PPG signal, a sensor that has measured a test PPG signal having a highest signal quality among a plurality of test PPG signals.

## Description

### Technical Field

The present disclosure relates to an atrial fibrillation determination system. More specifically, the present disclosure relates to a deep-learning-based atrial fibrillation determination system using a PPG (photoplethysmography) signal detection ring.

### Background Art

Atrial fibrillation is an arrhythmia that causes irregular heartbeat in the atria. Atrial fibrillation occurs intermittently, and early diagnosis has been difficult because a visit to the hospital is required for a diagnosis through an electrocardiogram (ECG), which is widely used to determine atrial fibrillation. However, with reliable continuous monitoring, the risk can be reduced by early detection and management of patients. Therefore, a system and method for monitoring atrial fibrillation in real time in daily life is needed.

### Disclosure

### Technical Problem

An objective of the present disclosure is to provide a deep-learning-based atrial fibrillation determination system by using a PPG signal detection ring.

### Technical Solution

In order to solve the objective described above, a deep-learning-based atrial fibrillation determination system using a PPG signal detection ring includes a server, wherein the server includes: a signal quality classification component configured to classify quality of a PPG signal as good or bad; and an atrial fibrillation determination component configured to determine, by using a deep learning model, from the PPG signal, whether atrial fibrillation has occurred, wherein the PPG signal is measured using the PPG signal detection ring and the server receives the PPG signal from the PPG signal detection ring through a terminal, the PPG signal detection ring includes a plurality of sensors configured to simultaneously measure a plurality of PPG signals at different locations, each of the plurality of sensors includes a light source and a photoelectric conversion device, and the terminal includes a sensor selection component configured to select, from among the plurality of sensors, as a sensor for measuring the PPG signal, a sensor that has measured a test PPG signal having a highest signal quality among a plurality of test PPG signals.

In some embodiments, signal quality of the plurality of test PPG signals may be evaluated based on at least one of magnitude of an acceleration signal, a signal-to-noise ratio, and a ratio of an AC component magnitude to a DC component magnitude.

In some embodiments, the server may further include an atrial fibrillation index calculation component configured to calculate an atrial fibrillation index, and the atrial fibrillation index may be defined by a ratio between a time during which the quality of the PPG signal is classified as good by the signal quality classification component and a time during which the quality of the PPG signal is classified as good by the signal quality classification component and it is determined by the atrial fibrillation determination component that atrial fibrillation has occurred.

In some embodiments, the terminal may further include a light source control component configured to control the light source of each of the plurality of sensors such that a DC component of each of the plurality of test PPG signals measured using the plurality of sensors is within a predetermined range.

In some embodiments, light source control by the light source control component and sensor selection by the sensor selection component may be performed sequentially, and the light source control by the light source control component and the sensor selection by the sensor selection component may be performed periodically.

A deep-learning-based atrial fibrillation determination method using a PPG signal detection ring, includes: receiving a PPG signal from the PPG signal detection ring through a terminal; classifying quality of the PPG signal as good or bad; and determining, by using a deep learning model from the PPG signal, whether atrial fibrillation has occurred, wherein the PPG signal detection ring includes a plurality of sensors configured to simultaneously measure a plurality of PPG signals at different locations, and each of the plurality of sensors includes a light source and a photoelectric conversion device, the PPG signal is measured using the PPG signal detection ring, the server receives the PPG signal from the PPG signal detection ring through a terminal, and the terminal may include a sensor selection component configured to select, from among the plurality of sensors, as a sensor for measuring the PPG signal, a sensor that has measured a test PPG signal having a highest signal quality among a plurality of test PPG signals.

In some embodiments, signal quality of the plurality of test PPG signals may be evaluated based on at least one of magnitude of an acceleration signal, a signal-to-noise ratio, and a ratio of an AC component magnitude to a DC component magnitude.

In some embodiments, the method may further include calculating an atrial fibrillation index, wherein the atrial fibrillation index may be defined by a ratio between a time during which the quality of the PPG signal is classified as good by a signal quality classification component and a time during which the quality of the PPG signal is classified as good by the signal quality classification component and it is determined by the atrial fibrillation determination component that atrial fibrillation has occurred.

In some embodiments, the terminal may further include a light source control component configured to control the light source of each of the plurality of sensors such that a DC component of each of the plurality of test PPG signals measured using the plurality of sensors is within a predetermined range.

In some embodiments, light source control by the light source control component and sensor selection by the sensor selection component may be performed sequentially, and the light source control by the light source control component and the sensor selection by the sensor selection component may be performed periodically.

### Advantageous Effects

A deep-learning-based atrial fibrillation determination system using a PPG signal detection ring is provided. According to the present disclosure, atrial fibrillation may be continuously and easily monitored in daily life. According to the present disclosure, the PPG signal detection ring may include a plurality of sensors. Among the plurality of sensors, a sensor with a PPG signal having excellent signal quality may be selected. Therefore, a sensor may be selected to suit the location and shape of blood vessels that are different for each user.

### Description of Drawings

FIG. 1 is a three-dimensional diagram of a PPG signal detection ring according to an embodiment of the present disclosure.
FIG. 2 is an exploded three-dimensional view of a PPG signal detection ring according to an embodiment of the present disclosure.
FIG. 3 is a block diagram of a deep-learning-based atrial fibrillation determination system using a PPG signal detection ring according to an embodiment of the present disclosure.
FIGS. 4A and 4B are graphs showing PPG signals before preprocessing.
FIGS. 4C and 4D are graphs showing the PPG signals of FIGS. 4A and 4B after preprocessing, respectively.
FIGS. 5A and 5B are graphs showing PPG signals classified as good quality.
FIGS. 5C and 5D are graphs showing PPG signals classified as bad quality.
FIGS. 6A and 6B are graphs showing PPG signals determined not to be atrial fibrillation.
FIGS. 6C and 6D are graphs showing PPG signals determined to be atrial fibrillation.
FIG. 7 is a flowchart of a deep-learning-based atrial fibrillation determination method using a PPG signal detection ring.
FIG. 8 is a flowchart of a method of setting a sensor of a PPG signal detection ring.

### Best Mode

### Mode for Invention

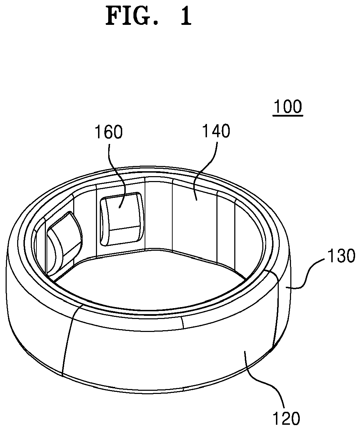

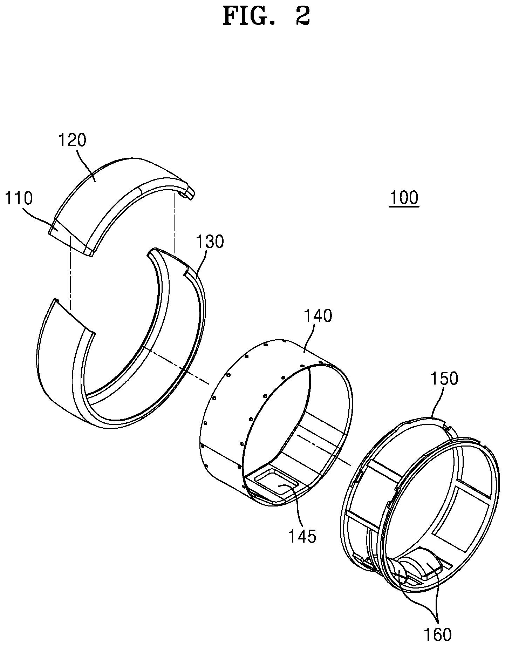

FIG. 1 is a three-dimensional diagram of a PPG signal detection ring 100 according to an embodiment of the present disclosure. FIG. 2 is an exploded three-dimensional view of the PPG signal detection ring 100 according to an embodiment of the present disclosure.

Referring to FIGS. 1 and 2, the PPG signal detection ring 100 may include an external electrode 130, an internal electrode 140, an insulating unit 150, a top cover 110, an operation display unit 120, and a plurality of sensors 160.

The external electrode 130 may have an arc shape. The external electrode 130 may include a conductor and may function as an electrode for measuring an electrocardiogram (ECG). Additionally, the external electrode 130 may form the appearance of the PPG signal detection ring 100, and the external electrode 130 may be in contact with the body of a user.

The internal electrode 140 may have a ring shape and may have a plurality of openings 145 for the plurality of sensors 160. The internal electrode 140 may include a conductor and may function as an electrode for measuring electrocardiogram. Additionally, the internal electrode 140 may form an inner tube of the PPG signal detection ring 100, and the internal electrode 140 may contact the finger of a user.

The insulating unit 150 may be disposed between the external electrode 130 and the internal electrode 140. The insulation unit 150 may enable electrical insulation between the external electrode 130 and the internal electrode 140.

The top cover 110 may have an arc shape and may form a ring shape together with the external electrode 130. The top cover 110 may form the appearance of the PPG signal detection ring 100.

The operation display unit 120 may be coupled to the top cover 110. The operation display unit 120 may include a plurality of LEDs, for example, a green LED and a red LED. The operation display unit 120 may display the operation of the PPG signal detection ring 100 by using a plurality of LEDs. For example, a green LED may be turned on for two seconds to indicate the start of measurement, and a green LED may be turned on for one second to indicate the end of measurement. Additionally, the red LED may be repeatedly turned on and off at 1-second intervals to notify of a malfunction.

The plurality of sensors 160 may be arranged to contact the finger of the user. The plurality of sensors 160 may each be located within the plurality of openings 145 of the internal electrode 140 and may protrude from a surface of the internal electrode 140. The plurality of sensors 160 may be configured to obtain different PPG signals at different locations. Each sensor 160 may include a light source and a photoelectric conversion device.

In some embodiments, although not illustrated in FIGS. 1 and 2, the PPG signal detection ring 100 may further include an acceleration sensor disposed between the external electrode 130 and the internal electrode 140.

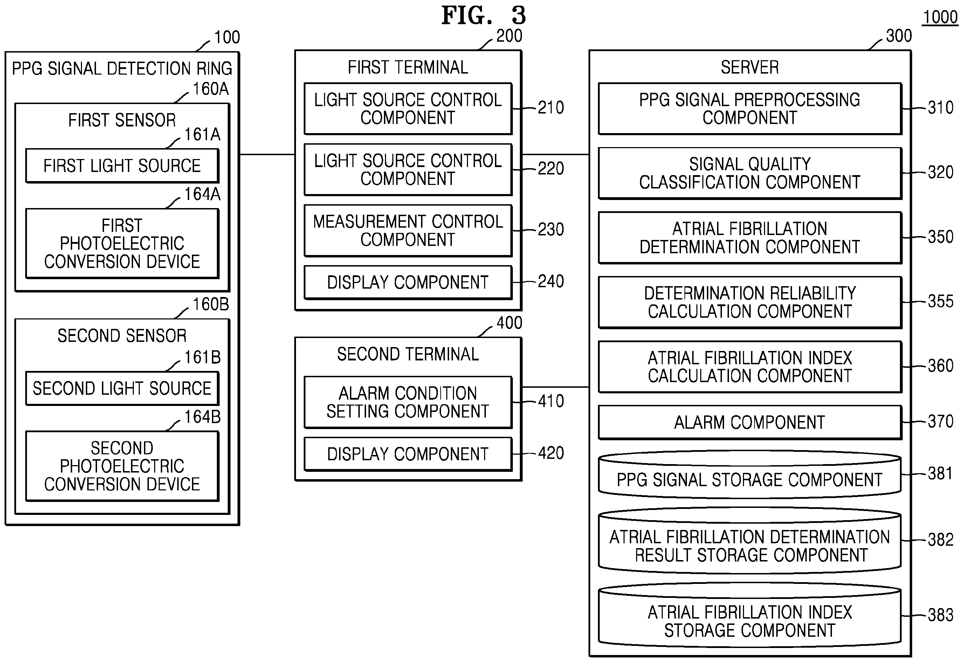

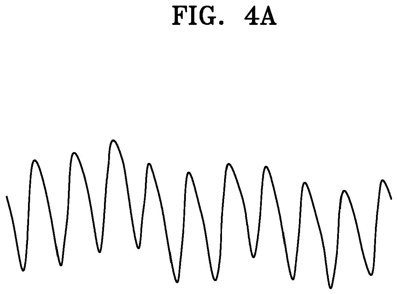

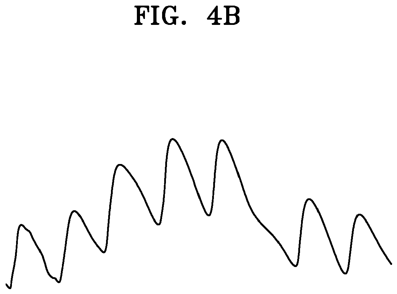

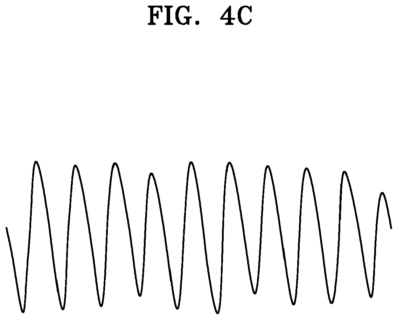

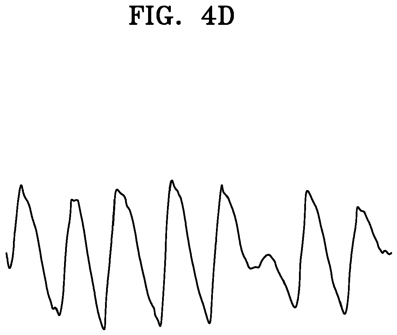

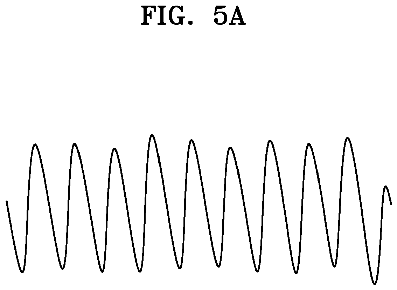

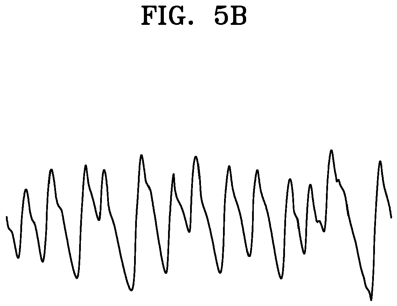

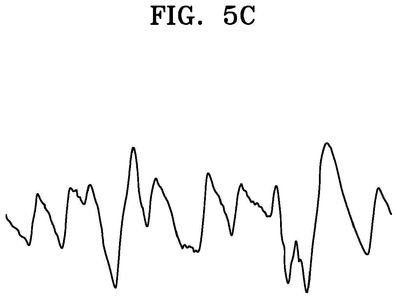

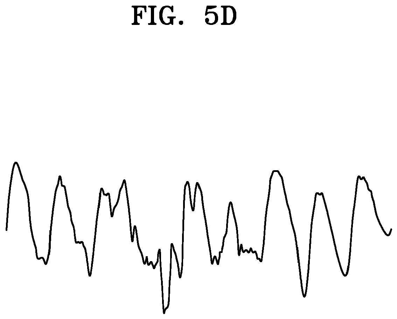

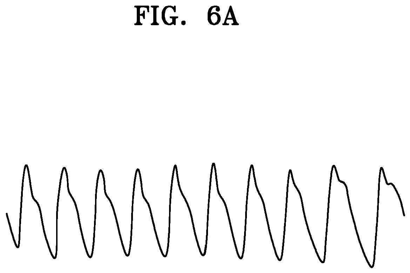

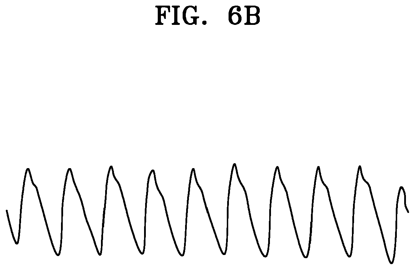

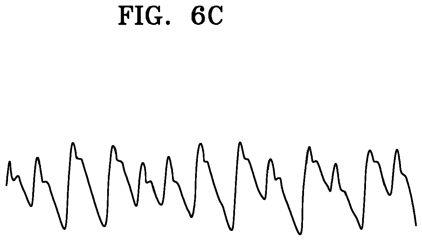

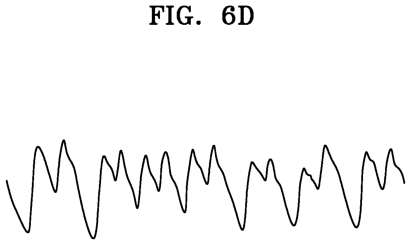

FIG. 3 is a block diagram of a deep-learning-based atrial fibrillation determination system 1000 using a PPG signal detection ring according to an embodiment of the present disclosure. FIGS. 4A and 4B are graphs showing PPG signals before preprocessing. FIGS. 4C and 4D are graphs showing the PPG signals of FIGS. 4A and 4B after preprocessing, respectively. FIGS. 5A and 5B are graphs showing PPG signals classified as good quality. FIGS. 5C and 5D are graphs showing PPG signals classified as bad quality. FIGS. 6A and 6B are graphs showing PPG signals determined not to be atrial fibrillation. FIGS. 6C and 6D are graphs showing PPG signals determined to be atrial fibrillation.

Referring to FIGS. 3, 4A to 4D, 5A to 5D, and 6A to 6D, the deep-learning-based atrial fibrillation determination system 1000 using a PPG signal detection ring may include a PPG signal detection ring 100, a first terminal 200, a server 300, and a second terminal 400.

The PPG signal detection ring 100 may include a plurality of sensors, for example, a first sensor 160A and a second sensor 160B. Although the PPG signal detection ring 100 is illustrated as including two sensors in FIG. 3, the number of sensors included in the PPG signal detection ring 100 is not limited to two. In some embodiments, although not illustrated in FIG. 3, the PPG signal detection ring 100 may further include an acceleration sensor.

The first sensor 160A may include a first light source 161A and a first photoelectric conversion device 164A. The first sensor 160A may detect a first PPG signal by using the first light source 161A and the first photoelectric conversion device 164A. The first light source 161A may include, for example, a green, red, or infrared LED, and the first photoelectric conversion device 164A may include a photo diode.

The second sensor 160B may include a second light source 161B and a second photoelectric conversion device 164B. The second sensor 160B may detect a second PPG signal by using the second light source 161B and the second photoelectric conversion device 164B. The second light source 161B may include, for example, a green, red, or infrared LED. The second light source 161B may emit light of the same wavelength as the first light source 161A. The second photoelectric conversion device 164B may include a photo diode.

The acceleration sensor may detect movement of the user by measuring acceleration of the PPG signal detection ring 100.

The first terminal 200 may be used by the user. The first terminal 200 may include, for example, a smartphone or a tablet PC. The first terminal 200 may be connected to the PPG signal detection ring 100 by wire or wirelessly. For example, the first terminal 200 may be connected to the PPG signal detection ring 100 using Bluetooth or Wi-Fi. The first terminal 200 may be connected to the server 300 by wire or wirelessly. For example, the first terminal 200 may be connected to the server 300 through Wi-Fi or mobile telecommunication technology such as 3G, LTE, or 5G.

The first terminal 200 may include a light source control component 210, a sensor selection component 220, a measurement control component 230, and a display component 240. An application may be installed in the first terminal 200. The light source control component 210, the sensor selection component 220, the measurement control component 230, and the display component 240 may be implemented by an application. Alternatively, the first terminal 200 may access a website, and the light source control component 210, the sensor selection component 220, the measurement control component 230, and the display component 240 may be implemented by the website.

The light source control component 210 may control the first light source 161A and the second light source 161B such that a DC component of each of the two test PPG signals received from the first sensor 160A and the second sensor 160B satisfies a predetermined range.

After the light source control component 210 controls the first light source 161A and the second light source 161B, the sensor selection component 220 may select one of the first sensor 160A and the second sensor 160B as a sensor to measure a PPG signal. The sensor selection component 220 may select a sensor that has measured a test PPG signal having a highest signal quality among two test PPG signals from the first sensor 160A and the second sensor 160B among the first sensor 160A and the second sensor 160B. Signal quality may be evaluated by at least one of the magnitude of an acceleration signal, a signal to noise ratio (SNR), and a ratio of an AC component magnitude to a DC component magnitude. The smaller the acceleration signal, the higher the signal-to-noise ratio, and the higher the ratio of the AC component magnitude to the DC component magnitude, the higher the signal quality may be. A PPG signal may then be measured from a sensor selected by the sensor selection component 220.

Control of the first light source 161A and the second light source 161B by the light source control component 210 and sensor selection by the sensor selection component 220 may be performed sequentially. That is, after the light source control component 210 controls the first light source 161A and the second light source 161B, the sensor selection component 220 may select a sensor. The control of the first light source 161A and the second light source 161B by the light source control component 210 and the sensor selection by the sensor selection component 220 may be performed periodically. The PPG signal detection ring 100 may move or rotate relative to the finger, and thus, the light source control component 210 may periodically control the light sources and the sensor selection component 220 may periodically select a sensor.

The measurement control component 230 may select a measurement mode or start or end measurement according to a user input. For example, the user may select a measurement mode between a self-check mode and a background mode by using the measurement control component 230. In the self-check mode, the user may start and end measurement by using the measurement control component 230. In the background mode, measurement starts and continues regardless of a user input. In the background mode, the user may set or change a measurement cycle. In some embodiments, the measurement control component 230 may be included in the PPG signal detection ring 100. That is, the user may select a measurement mode or start or end a measurement by using the measurement control component 230 of the PPG signal detection ring 100. In some embodiments, the measurement control component 230 may be included in the server 300. That is, a service provider may select a measurement mode or start or end measurement by using the measurement control component 230 of the server 300.

The display component 240 may display at least one of a PPG signal measured by the PPG signal detection ring 100 and stored in a PPG signal storage component 381 of the server 300, a measurement date and time of the PPG signal stored in the PPG signal storage component 381 of the server 300, a signal quality classification result classified by a signal quality classification component 320 of the server 300 and stored in an atrial fibrillation determination result storage component 382 of the server 300, an atrial fibrillation determination result determined by an atrial fibrillation determination component 350 of the server 300 and stored in the atrial fibrillation determination result storage component 382 of the server 300, a determination reliability calculated by a determination reliability calculation component 355 of the server 300 and stored in the atrial fibrillation determination result storage component 382 of the server 300, and an atrial fibrillation index calculated by an atrial fibrillation index calculation component 360 of the server 300 and stored in an atrial fibrillation index storage component 383.

The server 300 may include a PPG signal preprocessing component 310, the signal quality classification component 320, the atrial fibrillation determination component 350, the determination reliability calculation component 355, the PPG signal storage component 381, and the atrial fibrillation determination result storage component 382. In some embodiments, the server 300 may further include the atrial fibrillation index calculation component 360 and the atrial fibrillation index storage component 383. In some embodiments, the server 300 may further include an alarm component 370.

The PPG signal preprocessing component 310 may preprocess a PPG signal received by the server 300 through the first terminal 200 from a selected sensor of the PPG signal detection ring 100. For example, low-pass filters, high-pass filters, and normalization may be used to preprocess the PPG signal. FIGS. 4A and 4B illustrate PPG signals before preprocessing, and FIGS. 4C and 4D illustrate the PPG signal of FIGS. 4A and 4B, respectively, after preprocessing. In some embodiments, the PPG signal preprocessing component 310 may preprocess an acceleration signal received by the server 300 through the first terminal 200 from the acceleration sensor of the PPG signal detection ring 100.

The signal quality classification component 320 may classify the signal quality of a PPG signal as good or bad. FIGS. 5A and 5B illustrate PPG signals classified as good quality by the signal quality classification component 320. FIGS. 5C and 5D illustrate PPG signals classified as bad quality by the signal quality classification component 320. In some embodiments, the signal quality classification component 320 may refer to the acceleration signal to classify the signal quality of the PPG signal as either good or bad.

An atrial fibrillation determination result from a PPG signal that is classified as bad quality may be determined to be unreliable and may not be displayed by the display component 240 of the first terminal 200. An atrial fibrillation determination result from the PPG signal that is classified as good quality may be determined to be reliable and may be displayed by the display component 240 of the first terminal 200. In an alternative embodiment, an atrial fibrillation determination result from a PPG signal may be displayed by the display component 240 of the first terminal 200 together with a signal quality classification result, regardless of the signal quality classification result. Additionally, regardless of the signal quality classification result, the atrial fibrillation determination result from the PPG signal may be displayed by a display component 420 of the second terminal 400 together with the signal quality classification result.

In some embodiments, the signal quality classification component 320 may classify the quality of a PPG signal as good or bad by using a deep learning model. In some embodiments, the signal quality classification component 320 may refer to an acceleration signal to classify the quality of the PPG signal as good or bad. The deep learning model used in the signal quality classification component 320 may include convolution neural network (CNN), Long Short-Term Memory (LSTM), Fully Connected Network (FCN), an encoder, a decoder, or a combination thereof. Methods for classifying signal quality are not limited to those described herein. The atrial fibrillation determination component 350 may determine whether atrial fibrillation has occurred, from a PPG signal by using a deep learning model. FIGS. 6A and 6B are graphs in which it is determined by the atrial fibrillation determination component 350 that atrial fibrillation has not occurred. FIGS. 6C and 6D are graphs in which it is determined by the atrial fibrillation determination component 350 that atrial fibrillation has occurred. The atrial fibrillation determination component 350 may calculate a probability of occurrence of atrial fibrillation, from a PPG signal, and determine that atrial fibrillation has occurred when the probability of occurrence of atrial fibrillation exceeds a threshold (for example, 0.5), and determine that no atrial fibrillation has occurred if the probability of occurrence of atrial fibrillation is not greater than the threshold (for example, 0.5).

A deep learning model used in the atrial fibrillation determination component 350 may include CNN, LSTM, FCN, encoder, decoder, or a combination thereof. Deep learning models may be trained using supervised learning. Data augmentation may be used to ensure a sufficient data set for supervised learning.

The determination reliability calculation component 355 may calculate a reliability of atrial fibrillation determination. The determination reliability calculation component 355 may calculate a determination reliability, for example, by applying temperature scaling to the atrial fibrillation determination probability calculated by the atrial fibrillation determination component 350. (Guo C, Pleiss G, Sun Y, Weinberger KQ, "On Calibration of Modern Neural Networks", Proceedings of the 34th International Conference on Machine Learning, PMLR 70, 1321-1330, 2017)

The atrial fibrillation index calculation component 360 may calculate an atrial fibrillation index. The atrial fibrillation index may be defined based on a time during which the quality of a PPG signal is classified as good by the signal quality classification component 320 and a time during which it is determined by the atrial fibrillation determination component 350 that atrial fibrillation has occurred while the quality of a PPG signal is classified as good by the signal quality classification component 320. For example, the atrial fibrillation index may be defined by a ratio between a time during which the quality of a PPG signal is classified as good by the signal quality classification component 320 and a time during which it is determined by the atrial fibrillation determination component 350 that atrial fibrillation has occurred while the quality of a PPG signal is classified as good by the signal quality classification component 320. In other words, the atrial fibrillation index may refer to a ratio of the time when atrial fibrillation occurred with respect to times at which a reliable atrial fibrillation determination result may be obtained.

When at least one of the atrial fibrillation determination result determined by the atrial fibrillation determination component 350 and the atrial fibrillation index calculated by the atrial fibrillation index calculation component 360 satisfies an alarm condition set by an alarm condition setting component 410 of the second terminal 400, the alarm component 370 may transmit an alarm to at least one of the first terminal 200 and the second terminal 400.

The PPG signal storage component 381 may store the PPG signal received by the server 300 through the first terminal 200 from a selected sensor of the PPG signal detection ring 100. The PPG signal storage component 381 may further store a measurement date and time of the PPG signal. The PPG signal storage component 381 may further store a PPG signal preprocessed by the PPG signal preprocessing component 310. The PPG signal storage component 381 may further store an acceleration signal received by the server 300 from the acceleration sensor of the PPG signal detection ring 100 through the first terminal 200. The PPG signal storage component 381 may further store an acceleration signal preprocessed by the PPG signal preprocessing component 310. The atrial fibrillation determination result storage component 382 may store the atrial fibrillation determination result determined by the atrial fibrillation determination component 350. The atrial fibrillation determination result storage component 382 may further store a signal quality classification result classified by the signal quality classification component 320 and a determination reliability calculated by the determination reliability calculation component 355. The atrial fibrillation index storage component 383 may store the atrial fibrillation index calculated by the atrial fibrillation index calculation component 360.

The second terminal 400 may be connected to the server 300 by wire or wirelessly. For example, the second terminal 200 may be connected to the server 300 through Wi-Fi or mobile telecommunication technology such as 3G, LTE, or 5G. The second terminal 400 may be used by a doctor. The second terminal 400 may include, for example, a smartphone, tablet PC, computer, or laptop.

The second terminal 400 may include the alarm condition setting component 410 and the display component 420. The second terminal 400 may be connected to a website or have an application installed. The alarm condition setting component 410 and the display component 420 may be implemented using a website or application.

Using the alarm condition setting component 410, the doctor may set alarm conditions. For example, an alarm may be set to sound if atrial fibrillation continues for 10 minutes or more.

The display component 420 may display at least one of a PPG signal from the PPG signal storage component 381, a measurement date and time of the PPG signal stored in the PPG signal storage component 381 of the server 300, a preprocessed PPG signal stored in the PPG signal storage component 381, a signal quality classification result from the atrial fibrillation determination result storage component 382, an atrial fibrillation determination result from the atrial fibrillation determination result storage component 382, a determination reliability from the atrial fibrillation determination result storage component 382, and an atrial fibrillation index from the atrial fibrillation index storage component 383.

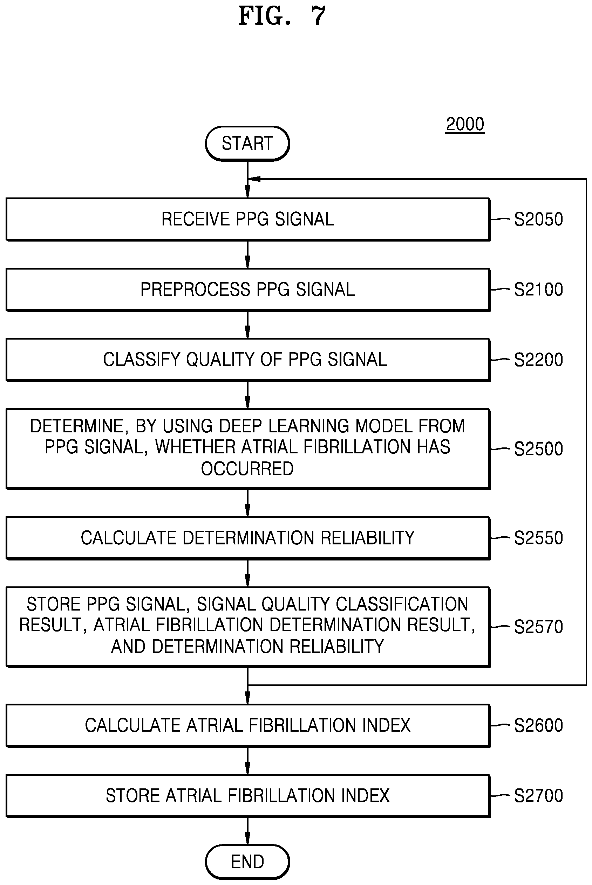

FIG. 7 is a flowchart of a deep-learning-based atrial fibrillation determination method 2000 using a PPG signal detection ring.

Referring to FIGS. 3 and 7, the server 300 may receive a PPG signal from a selected sensor of the PPG signal detection ring 100 through the first terminal 200 (S2050). Next, the PPG signal preprocessing component 310 may preprocess the PPG signal (S2100). For example, low-pass filters, high-pass filters, and normalization may be used to preprocess the PPG signal. FIGS. 4A and 4B illustrate PPG signals before preprocessing, and FIGS. 4C and 4D illustrate the PPG signals of FIGS. 4A and 4B, respectively, after preprocessing. In some embodiments, the PPG signal preprocessing component 310 may further preprocess the acceleration signal received by the server 300 through the first terminal 200 from the acceleration sensor of the PPG signal detection ring 100.

Next, the signal quality classification component 320 may classify the signal quality of the PPG signal as good or bad (S2200). FIGS. 5A and 5B illustrate PPG signals classified as good quality by the signal quality classification component 320. FIGS. 5C and 5D illustrate PPG signals classified as bad quality by the signal quality classification component 320. An atrial fibrillation determination result from the PPG signal classified as bad quality may be determined to be unreliable and may not be displayed by the display component 240 of the first terminal 200. An atrial fibrillation determination result from the PPG signal classified as good quality may be determined to be reliable and may be displayed by the display component 240 of the first terminal 200.

In some embodiments, a deep learning model may be used in the signal quality classification component 320. The deep learning model used in the signal quality classification component 320 may include convolution neural network (CNN), Long Short-Term Memory (LSTM), Fully Connected Network (FCN), an encoder, a decoder, or a combination thereof. Methods for classifying signal quality are not limited to those described herein. Next, the atrial fibrillation determination component 350 may determine, by using a deep learning model from the PPG signal, whether atrial fibrillation has occurred (S2500). FIGS. 6A and 6B are graphs in which it is determined by the atrial fibrillation determination component 350 that atrial fibrillation has not occurred. FIGS. 6C and 6D are graphs in which it is determined by the atrial fibrillation determination component 350 that atrial fibrillation has occurred. The atrial fibrillation determination component 350 may calculate a probability of occurrence of atrial fibrillation, from a PPG signal, and determine that atrial fibrillation has occurred when the probability of occurrence of atrial fibrillation exceeds a threshold (for example, 0.5), and determine that no atrial fibrillation has occurred if the probability of occurrence of atrial fibrillation is not greater than the threshold (for example, 0.5).

A deep learning model used in the atrial fibrillation determination component 350 may include CNN, LSTM, FCN, encoder, decoder, or a combination thereof. The deep learning model may be trained using supervised learning. Data augmentation may be used to ensure a sufficient data set for supervised learning.

Next, the determination reliability calculation component 355 may calculate a reliability of the atrial fibrillation determination (S2550). The determination reliability calculation component 355 may calculate a determination reliability, for example, by applying temperature scaling to the atrial fibrillation determination probability calculated by the atrial fibrillation determination component 350. (Guo C, Pleiss G, Sun Y, Weinberger KQ, "On Calibration of Modern Neural Networks", Proceedings of the 34th International Conference on Machine Learning, PMLR 70, 1321-1330, 2017)

Next, the PPG signal, the signal quality classification result, the atrial fibrillation determination result, and the determination reliability may be stored (S2570). The PPG signal may be stored in the PPG signal storage component 381. The atrial fibrillation determination result, the signal quality classification result, and the determination reliability may be stored in the atrial fibrillation determination result storage component 382.

Next, an operation of receiving a PPG signal (S2050), an operation of preprocessing the PPG signal (S2100), an operation of classifying the quality of the PPG signal (S2220), an operation of determining whether atrial fibrillation has occurred (S2500), an operation of calculating a determination reliability (S2550), and an operation of storing the PPG signal, a signal quality classification result, an atrial fibrillation determination result, and the determination reliability (S2570) may be repeated.

Next, the atrial fibrillation index calculation component 360 may calculate an atrial fibrillation index (S2600). The atrial fibrillation index may be defined based on a time during which the quality of the PPG signal is classified as good by the signal quality classification component 320 and the time during which the quality of the PPG signal is classified as good by the signal quality classification component 320 and which is determined as a time during which atrial fibrillation has occurred, by the atrial fibrillation determination component 350. For example, the atrial fibrillation index may be defined by a ratio between the time during which the quality of the PPG signal is classified as good by the signal quality classification component 320 and the time during which the quality of the PPG signal is classified as good by the signal quality classification component 320 and it is determined by the atrial fibrillation determination component 350 that atrial fibrillation has occurred. In other words, the atrial fibrillation index may refer to the time when atrial fibrillation occurred among the times when a reliable atrial fibrillation determination result may be obtained.

Next, the atrial fibrillation index may be stored in the atrial fibrillation index storage component 383 (S2700).

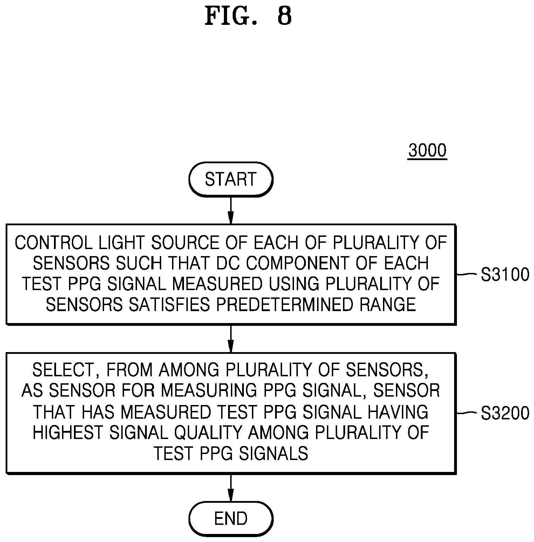

FIG. 8 is a flowchart of a method 3000 of setting a sensor of a PPG signal detection ring.

Referring to FIGS. 8 and 3, the light source control component 210 may control each light source such that a DC component of each test PPG signal measured using a plurality of sensors satisfies a predetermined range (S3100). Operation S3100 is to set each sensor, for example, the first sensor 160A and the second sensor 160B, to the best state for measuring a PPG signal.

Next, the sensor selection component 220 may select one of the plurality of sensors as a sensor for later measuring a PPG signal (S3200). The sensor selection component 220 may select a sensor that has measured a test PPG signal having a highest signal quality among two test PPG signals from the first sensor 160A and the second sensor 160B among the first sensor 160A and the second sensor 160B. Signal quality may be evaluated by at least one of the magnitude of an acceleration signal, a signal-to-noise ratio, and a ratio of the AC component magnitude to the DC component magnitude. The smaller an acceleration signal, the higher the signal-to-noise ratio, and the higher the ratio of the AC component magnitude to the DC component magnitude, the higher the signal quality may be. Thereafter, a PPG signal measured from the sensor selected by the sensor selection component 220 may be transmitted to the server 300 through the first terminal 200.

Operation of controlling a light source of each of the plurality of sensors (S3100) and operation of selecting a sensor (S3200) may be performed sequentially. Additionally, the method 3000 of setting a sensor of a PPG signal detection ring may be performed periodically. The PPG signal detection ring 100 may move or rotate relative to the finger, and thus, the light source control component 210 may periodically control the light sources and the sensor selection component 220 may periodically select a sensor.

The embodiments disclosed in the present disclosure are not intended to limit the technical idea of the present disclosure, but are for illustrative purposes, and the scope of the technical idea of the present disclosure is not limited by these embodiments. The scope of protection of the present disclosure should be interpreted in accordance with the claims below, and all technical ideas within the equivalent scope should be interpreted as being included in the scope of rights of the present disclosure.

## Claims

1. A deep-learning-based atrial fibrillation determination system using a photoplethysmography (PPG) signal detection ring, the system comprising a server, wherein the server comprises:
a signal quality classification component configured to classify quality of a PPG signal as good or bad; and
an atrial fibrillation determination component configured to determine, by using a deep learning model, from the PPG signal, whether atrial fibrillation has occurred,
wherein the PPG signal is measured using the PPG signal detection ring, and the server receives the PPG signal from the PPG signal detection ring through a terminal,
the PPG signal detection ring includes a plurality of sensors configured to simultaneously measure a plurality of PPG signals at different locations,
each of the plurality of sensors includes a light source and a photoelectric conversion device, and the terminal includes a sensor selection component configured to select, from among the plurality of sensors, as a sensor for measuring the PPG signal, a sensor that has measured a test PPG signal having a highest signal quality among a plurality of test PPG signals.

2. The system of claim 1, wherein signal quality of the plurality of test PPG signals is evaluated based on at least one of magnitude of an acceleration signal, a signal-to-noise ratio, and a ratio of an AC component magnitude to a DC component magnitude.

3. The system of claim 1, wherein the server further comprises an atrial fibrillation index calculation component configured to calculate an atrial fibrillation index, and
the atrial fibrillation index is defined by a ratio between a time during which the quality of the PPG signal is classified as good by the signal quality classification component and a time during which it is determined by the atrial fibrillation determination component that atrial fibrillation has occurred while the quality of a PPG signal is classified as good by the signal quality classification component.

4. The system of claim 1, wherein the terminal further comprises a light source control component configured to control the light source of each of the plurality of sensors such that a DC component of each of the plurality of test PPG signals measured using the plurality of sensors is within a predetermined range.

5. The system of claim 4, wherein light source control by the light source control component and sensor selection by the sensor selection component are performed sequentially, and
the light source control by the light source control component and the sensor selection by the sensor selection component are performed periodically.

6. A deep-learning-based atrial fibrillation determination method using a photoplethysmography (PPG) signal detection ring, the method comprising: receiving a PPG signal from the PPG signal detection ring through a terminal;
classifying quality of the PPG signal as good or bad; and
determining, by using a deep learning model from the PPG signal, whether atrial fibrillation has occurred,
wherein the PPG signal detection ring includes a plurality of sensors configured to simultaneously measure a plurality of PPG signals at different locations, and
each of the plurality of sensors includes a light source and a photoelectric conversion device, and the terminal includes a sensor selection component configured to select, from among the plurality of sensors, as a sensor for measuring the PPG signal, a sensor that has measured a test PPG signal having a highest signal quality among a plurality of test PPG signals.

7. The method of claim 6, wherein signal quality of the plurality of test PPG signals is evaluated based on at least one of magnitude of an acceleration signal, a signal-to-noise ratio, and a ratio of an AC component magnitude to a DC component magnitude.

8. The method of claim 6, further comprising calculating an atrial fibrillation index,
wherein the atrial fibrillation index is defined by a ratio between a time during which the quality of the PPG signal is classified as good by a signal quality classification component and a time during which it is determined by the atrial fibrillation determination component that atrial fibrillation has occurred while the quality of a PPG signal is classified as good by the signal quality classification component.

9. The method of claim 6, wherein the terminal further comprises a light source control component configured to control the light source of each of the plurality of sensors such that a DC component of each of the plurality of test PPG signals measured using the plurality of sensors is within a predetermined range.

10. The method of claim 9, wherein light source control by the light source control component and sensor selection by the sensor selection component are performed sequentially, and
the light source control by the light source control component and the sensor selection by the sensor selection component are performed periodically.
